Europäisches Patentamt

European Patent Office

Office européen des brevets

(11) Publication number: **0 239 605 B1**

# EUROPEAN PATENT SPECIFICATION

(45) Date of publication of patent specification: **08.07.92**

(51) Int. Cl.5: **A61K 9/24**, A61K 9/52, G03F 7/26

(21) Application number: **86905895.8**

(22) Date of filing: **02.10.86**

(86) International application number: **PCT/GB86/00594**

(87) International publication number: **WO 87/01937 (09.04.87 87/08)**

(54) STRUCTURES FOR CONTROLLED CHEMICAL RELEASE.

(30) Priority: **02.10.85 GB 8524369**

(43) Date of publication of application: **07.10.87 Bulletin 87/41**

(45) Publication of the grant of the patent: **08.07.92 Bulletin 92/28**

(84) Designated Contracting States: **BE CH DE FR GB IT LI NL SE**

(56) References cited: **US-A- 3 764 018**

(73) Proprietor: **The Secretary of State for Defence in Her Britannic Majesty's Government of the United Kingdom of Great Britain and Northern Ireland Whitehall London SW1A 2HB(GB)**

(72) Inventor: **MEARS, Adrian, Leonard 21 Collum End Rise Leckhampton Cheltenham Worcester GL53 OPA(GB)**
Inventor: **BENJAMIN, John, David 20 Britten Drive Poolbrook Malvern Worcester WR14 3LG(GB)**

(74) Representative: **Beckham, Robert William et al Procurement Executive Ministry of Defence Patents 1A(4), Room 2014 Empress State Building Lillie Road London SW6 1TR(GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services

## Description

For many applications it is desirable to obtain sustained and predictable release of a chemical. This is especially true in the case of drugs where it is desirable to maintain a well-controlled and stable release rate while avoiding the need for regular administration of the drug. There are three principal techniques for obtaining sustained release. The first is to release the material through an orifice, eg a needle valve or the tap of a burette. This can be mechanically complicated, especially for implanted drug release structures. The second uses diffusion through a membrane, eg using patches containing the drug which are affixed to the outside of the skin. The drug perfuses through the skin at a steady rate. This is easily administered but duration and the range of possible drugs is limited. A third approach is to incorporate the drug in a matrix (usually a polymer) which is formed into a pellet and implanted surgically or by means of a hypodermic device. The chief problem is that the surface area of the pellet, and thus the release rate of the drug, decrease with time.

Larger devices of this nature may be made by mechanical shaping, machinery or moulding. Thus, if the substance is in the form of a rod or wire coated with a protective layer but open at one or both ends, a constant release rate will be achieved. If the diameter of the rod or wire varies along its length, a time-dependent release will be achieved. One example of this is for cattle to be given slugs of magnesium which is placed in their rumen in order to prevent a deficiency disease known as 'the staggers' in the spring. A structure which utilises material more economically could be made by forming the slugs of metal by moulding, casting or rolling and cutting, followed by injection moulding of plastic round the outside leaving one or both of the ends open. An alternative to the use of plastic would be to sputter a ceramic layer, eg a few $\mu$ms of silicon nitride, again keeping one or both ends clear so that the metal can be dissolved out through them. Such techniques may not, however, be adapted readily to the production of very small structures which have a wide range of applications.

This invention therefore consists of a method of fabricating a structure for the controlled release of a substance located in the structure between two relatively insoluble layers, characterised by the following stages:

(a) depositing a first layer of a relatively insoluble material on a substrate whose top layer at least is a substance which can be dissolved without affecting the materials of which the structure is made;

(b) shaping the first layer of material by photolithography and etching;

(c) depositing a layer of the substance to be released on said first layer;

(d) shaping the layer of the substance by photolithography and etching;

(e) depositing a second layer of a relatively insoluble material over the layer of the substance;

(f) shaping said second layer by photolithography and etching; and

(g) removing the substrate by dissolving at least the top layer thereof,

the shapes of the layers being determined so that the soluble layer is entirely surrounded by the relatively insoluble layers except for one or more release orifices.

In one application of the invention, the said substance is in direct contact with the outside through the orifice in the structure, so that on coming into contact with a solvent, the substance is released through the orifice at a rate proportional to the surface area in contact with the solvent. By suitable design of the area in which the substance is deposited, the rate of release may be varied as a function of time.

In a second application of the invention, a layer of a different material of known solubility is interposed between the two insoluble layers, the said material interconnecting the orifice with one or more substances whose release is to be controlled, whereby a delayed and/or alternating release may be obtained.

Conveniently, a plurality of such structures may be fabricated on a single substrate and by layer processing technology a large number of very small structures may be produced. In all cases, the substance nay be formed in a matrix the breakdown of which will release the substance.

By way of example, a number of specific structures incorporating the invention, together with their means of manufacture and mode of application, will now be described with reference to the drawings, of which

Figures 1 and 2 are respectively longitudinal vertical and horizontal schematic sections of a basic structure for the controlled release of a substance,

Figure 3 is a corresponding horizontal section through a structure in which the rate of release of the substance is periodic,

Figure 4 is a longitudinal vertical section through a structure in which two different substances are released alternately,

Figures 5, 6 and 7 illustrate in detail three steps in the fabrication of a structure such as that illustrated in Figure 4,

Figure 8 illustrates a small structure which is designed to release a drug a certain time after application,
Figure 9 is a plan view of a device actuated by the receipt of a radio signal,
Figures 10 and 11 are sections at slightly enlarged scales of the device illustrated in Figure 9 along the lines 10-10 and 11-11 respectively, and
Figure 12 illustrates schematically equipment incorporating the invention for performing chemical assays.

Example 1

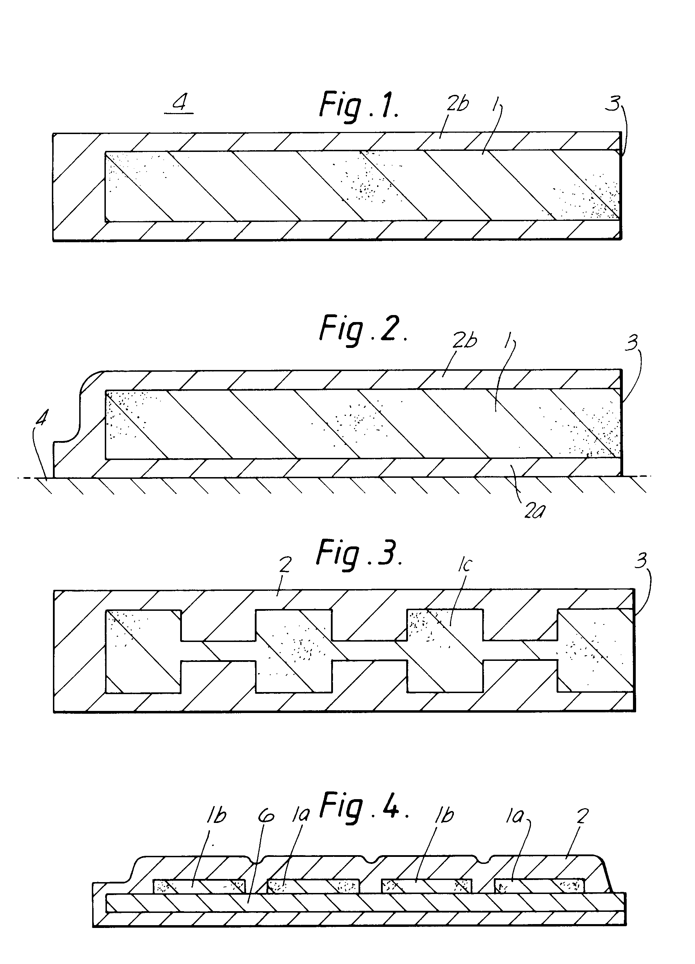

Figures 1 to 3 illustrate schematically the simplest structure according to the invention and suitable for the controlled release of drugs, in which a reservoir of material 1a containing the drug is sandwiched between two layers 2a, 2b of a relatively insoluble material. The shape of the reservoir layer 1 can be chosen so as to achieve whatever is desired as a function of time, but in general it contains the drug incorporated in a slowly-soluble material and is surrounded entirely, except at one edge 3, by a layer or layers 2a, 2b of a relatively insoluble material.

The reservoir 1 in the structure of Figures 1 and 2 can be folded back on itself or into a spiral, giving a much longer drug release period for a device of a given size and given rate of dissolution of the material in which the drug is impregnated. By the use of a suitably shaped reservoir 1c (see Figure 3), the cross-sectional area of the layer presented to the solvent, and hence the rate of dissolution, can be made to vary as dissolution progresses.

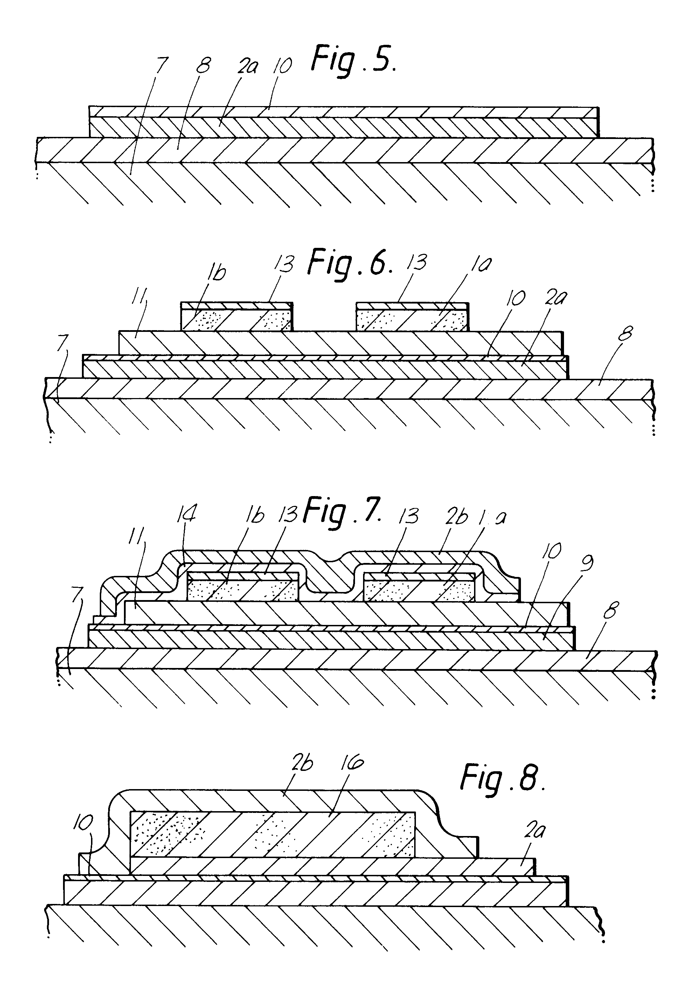

By the use of material impregnated with different drugs 1a and 1b in the same device (see Figure 4), coordinated sequential release of drugs can be achieved, and the periodicity may be controlled by the solubility of the connecting layer 6. If desired, a simultaneous release of more than one drug can be achieved.

For many drugs a constant rate of release is desirable, though in other cases, notably hormones (eg for birth control applications) a periodically-varying release is preferable. If the material is to be used inside the body it is best if the drug is incorporated into a polymer which dissolves into relatively small fragments, eg polyanhydrides. Other materials include polyethylene glycol and polyhydroxylethylmethyl methacrylate. Polymeric materials in which drugs may be absorbed for controlled release are reviewed in "Chemical and Physical Structure of Polymers as Carriers for Controlled Release of Bioactive Agents: A Review" by R Langer and N Peppas in J. Macromol.Sci. Rev.Macromol.Chem.Phys.(1983) C23 (1) pp61-126 Marcel Dekker Inc.

Such structures can be made by exploiting the layer processing technology used in the fabrication of integrated circuits, thin film and thick film hybrids and pcbs. The steps involved are:

1. Take a substrate 4 (see Figures 1 and 2) made of a material which can be dissolved without affecting the material of which the particles are to be made, or deposit a layer of such a material on top of a bulk substrate.

2. Deposit layers 2a, 1, 2b of the materials of which the particles are to be made on top of the layer. This may be done by sputtering, evaporation, chemical vapour deposition (CVD), metal-organic chemical vapour deposition (MOCVD) or dip coating.

3. Use photolithography (eg using optical, electron beam or ion beam lithography) followed by etching (plasma or wet chemical) to pattern the layers.

4. Dissolve the underlying substrate or soluble layer using a wet chemical process and filter out and wash the particles. If desired, more than one deposition, photolith and etch cycle may be used to make relatively complicated particles.

As an example of a more elaborate form of the invention, the structure illustrated in Figure 4 can be made by the following stages, with reference to Figures 5, 6 and 7:

1. Coat a silicon wafer 7 with 1 $\mu$m of silicon dioxide 8,

2. Add by dipping a covering layer of a relatively insoluble polymeric material 2a, eg rubber,

3. Sputter 0,1 $\mu$m (100 Å) of gold 10,

4. Carry out photolithography.

5. Etch gold using etch of 4 g KI, 1 g $I_2$, 40 ml $H_2O$ for about 10 s with resist from (4) as mask.

6. Plasma etch in an oxygen glow discharge with gold as a mask. This also burns off the resist. Equipment designed for plasma etching polyimide is suitable for this. The resulting structure is shown in Fig 5.

7. Dip coat with a material which is slowly soluble in blood with a uniform solution rate, eg a polyanhydride 11 (see Fig 6).

8. Pattern it by sputtering 0,1 $\mu$m (1000 Å) of aluminium, and after photolithography, plasma etch the aluminium using the resist mask and plasma etch the polyanhydride using the aluminium as a mask (burning off the resist at the same time).

9. Dissolve the aluminium in hydrochloric acid, optionally with a small addition of nitric acid.

10. Dip coat to deposit the first drug 1a in solid form or incorporated into a matrix so that it can be treated as a solid. The drug is typically incorporated into polylactic acid, polyglycolic acid or lactic/glycolic acid copolymer.

11. Pattern it in the same way as in (6).

12. Repeat steps 10 and 11 using the second drug 1b. The resulting structure is shown in Fig 6, a layer of aluminium 13 covering the drug deposits.

13. Sputter 0,1 $\mu$m (1000 Å) of gold 14.

14. Deposit a layer of the relatively insoluble material 2b, eg rubber, by dip coating and pattern it by the steps of 4-6 using a different mask.

15. Etch the gold with potassium iodide/iodine etch, with rubber acting as a mask. This results in the structure of Fig 7, which illustrates one only of a large number of devices formed on the same substrate 7.

16. Immerse in hydrofluoric acid which will dissolve the underlying silicon dioxide 8, thereby releasing the devices from the substrate 7. The top layer of aluminium is also removed in the same process.

The particles may if desired be filtered and washed, eg using millipore or nucleopore filters. If they are stored dry they can be re-suspended in saline by ultrasonification for 10 minutes.

An alternative route is to deposit 1 micron of aluminium in step 1, and an aluminium wet etch consisting of hydrochloric acid with a little nitric acid to release the particles from the substrate in step 16. This avoids any risk of fluoride ion incorporation into the structure in step 16.

In this structure the rubber layer provides protection and biocompatibility while the metal layers act as a diffusion barrier and ensure that the only way that the drug can escape is through the polyanhydride dissolving, this exposing the drugs which then also dissolve.

The use of layer processing to fabricate the particles has the advantages that:

1. The size and shape of the particles is accurately determined by physical (primarily lithographic) processes. It is also possible to make particles of more than one size and shape at the same time.

2. The number of particles produced in any batch is accurately controlled.

3. Relatively complicated particles can be made if desired.

4. The process is applicable to particles of a huge range of systems.

5. The equipment required is already in existence.

Possible applications of the structure include

1. Fertility control (using a long-lasting structure with pulsed release)

2. Release of growth hormone

3. Release of insulin in a steady rate (avoiding the fluctuations which arise from regular injections).

4. Release of vaccines, so that a series of doses can be delivered at intervals determined by the structure of the device.

5. Release of chemicals which produce a reduced sensitivity in the body to nerve agents.

6. Coordinated release of drugs. For example, a drug can be released which will cause cells to stop at a particular stage in their division cycle followed by another drug which will kill cells at this point. This is useful in cancer treatment.

Depending on the size of the devices, they may be placed in a body cavity, or injected (eg intramuscularly or subcutaneously) through a hypodermic needle. In the latter case, the use of a large number of small capsules (typically with a diameter of less than 500 microns) is necessary so they will go down the needle. Similar devices may be incorporated into coverings for burn wounds so as to release an antibiotic, eg penicillin, at a steady rate to prevent infection.

Example 2

Rather than depositing all the drug in the same place, it may be desirable to achieve distributed release throughout the body in order to avoid local concentration build-up. The drug can be incorporated into a large number of small particles of the type described in Example 1 which can be either inhaled or injected into the bloodstream or into body cavities joints, the genito-urinary tract or fluids eg the cerebro spinal fluid. The particles will then release their payloads in a predetermined way, controlled by the structure of the devices, over the extended region over which they are distributed.

Example 3

The use of particles similar to those described in Example 1 except in the respect that the particles carry a layer of antibody over part or all of their surface so that they stick to particular sites, eg tumours (in this case employing PLAP - placental alkaline phosphatase, HMFG - human milk fat globulin, CEA - Carcino-Embrionic antibody, HCG - Human Chorionic Gonadotrophin), so that these parts of the body receive more dosage than the rest. Details of techniques for coating solid surfaces with antibodies have been described by several writers.

In addition to direct coating of the surfaces, the surfaces can be coated by known techniques with a lipid layer to which antibodies are attached.

These particles need to be administered so that they are distributed over the region of interest and preferably so that they will move through it, thereby maximising the chance of their encountering the target tissue. In the case of injections into the bloodstream, the particles need to be minute (less than 5 microns in their maximum dimension) in order to go through capillaries, and it may be desirable just to perfuse them through the limb or organ of interest in a limited circulation, thereby avoiding the lungs, liver and spleen where they are liable to be trapped.

Example 4

The use of very small particles (typically less than 3 $\mu$m in diameter) in which the targeting is achieved by choice of particle size. In particular, devices can be made in which drug release will only occur after a certain delay. These particles may be injected into the blood and will be ingested by the reticuloendothelial cells. In due course the drug is released, treating those cells without needing to expose the rest of the body to the drug. This may be useful in the treatment of leishmaniasis, where a treatment based on the use of liposomes has proved successful. A typical design of particle is shown in Fig 8.

In this, a reservoir 1 of drug-containing material, 0·4 $\mu$m thick and 1 $\mu$m long, is deposited on a slowly-soluble layer 2a, 0·2 $\mu$m thick and longer in one direction by 0·7 $\mu$m. These two layers are formed as a thin gold layer 10 on the surface of a 0·1 $\mu$m thick insoluble layer. A top coating 2b of insoluble material, 0·1 $\mu$m thick, is formed over the reservoir 1 and extends 0·5 $\mu$m beyond the edge of the reservoir leaving the end 0·2 $\mu$m of the slowly-soluble layer 2a exposed. The slowly soluble material 2a therefore has to dissolve back 0·5 $\mu$m before the drug can be dissolved out, thereby providing the time delay.

Example 5

The release of drugs in parts of the body in which particular conditions, eg pH, are present are met by means of structures in which the drug is only exposed to the environment once a material which is only soluble in the particular conditions has dissolved. Apart from this requirement, the structures are the same as those of Figs 1 to 4 and the slowly-soluble polymer may be chosen to be soluble only under the appropriate conditions. Polymers whose solution rate is strongly pH-dependent are particularly suitable.

Example 6

The combining of chemical release with radioactive output, either for radiotherapy or to provide a marker of the position of the devices or of the part of the device which is labelled. To achieve this the radioisotope must be incorporated into the device. This may be achieved either by using materials which are radioactive when the device is made or by making the device using non-radio-active materials and after separating the devices from the substrate, washing and filtering them, then neuton-activating them. In either case, the radioactive or neutron activated species may be incorporated directly in one of the layers, or inserted by ion implantation.

For combining radiotherapy with chemotherapy, eg against cancer, antibody-labelled devices can be employed which release chemicals and also, for example, contain polonium 210. This is a pure alpha emitter so only the immediate surrounding tissue is irradiated.

For radiolabelling a gamma-emitter is used. One example of the incorporation of a gamma emitter is to implant $2 \times 10^{16}$ cm$^{-2}$ arsenic 75 ions into the devices and to perform the neutron activation at a flux of $5 \times 10^{12}$ neutrons cm$^{-2}$s$^{-1}$ for 24 hours. This will transmute roughly 1 atom in $10^{6}$ of the arsenic 75 to arsenic 76 which is a gamma emitter with a half life of 26.5 hours. Other nuclei which are also suitable for neutron activation into gamma emitters are tabulated below.

| Nuclei for neutron activation | | | | | |
|---|---|---|---|---|---|
| Nucleus | half life | gamma energy/keV | nucleus activated | (activity per gm)/Ci 7 days | after 23 days |
| As 76 | 26.3 h | 560 | As 75 | 4.6 | 4.6 |
| Au 198 | 2.7 d | 412 | Au | 33 | 40 |
| Lu 177 | 6.7 d | 208, 133. | Lu | 23.5 | 42 |
| Ir 192 | 74 d | 216, 468. | Ir | 10 | 31 |
| Sb 122 | 2.7 d | 564 | Sb | 2.1 | 2.6 |
| Sc 46 | 84 d | 889, 1121. | Sc | 2.2 | 7 |
| Yb 169 | 30.7 d | 63, 200 | Yb | 1.08 | 2.75 |
| Cr 51 | 27.7 d | 320 | Cr | 0.167 | 0.455 |
| Eu 152 | 13 y | wide range | Eu | 1.9 | 6.3 |

Example 7

Combining drug release with a label to make it easier to see where the particles have reached. In particular, the particles can incorporate a layer of a heavy element eg lead, platinum or gold (typically also acting as a relatively insoluble layer or diffusion barrier in a structure of the type shown in Figs 7 and 8) to enhance the particles' X-ray absorption. Alternative labels are fluorescence, and elements which provide nuclear magnetic resonance and electron spin resonance signals.

Example 8

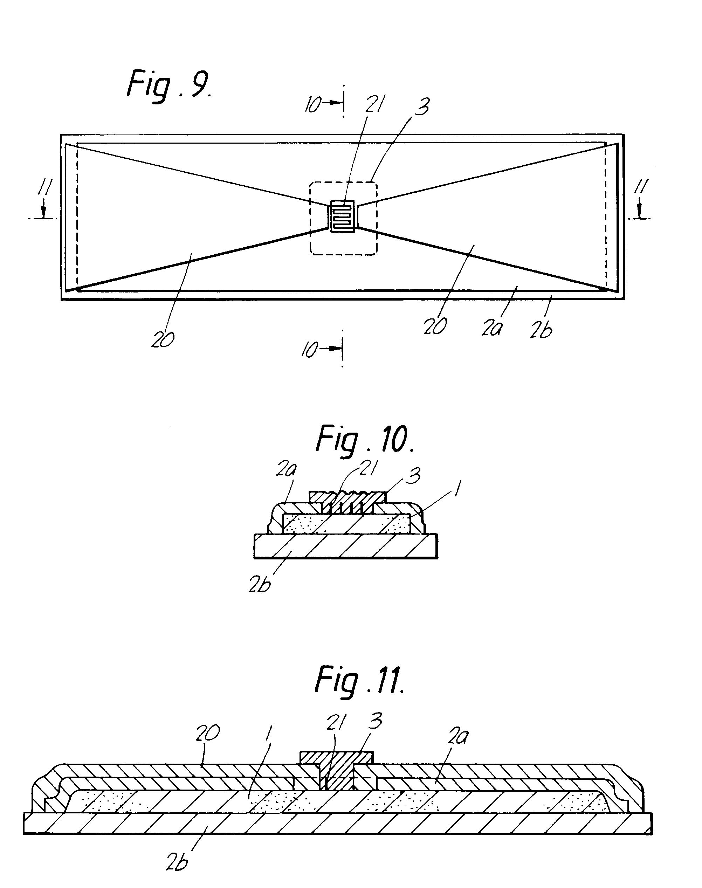

Combining drug release with a structure such as an aerial or piezoelectric transducer with a resistive load which will result in preferential energy deposition in the device when power in the form of alternating electric or magnetic fields, rf, microwaves or ultrasound are applied to the patient. The heating effect might be used simply to produce an increase in body temperature immediately around the devices, so as to speed up chemical reactions in that area. A more sophisticated application is embodied in the structure illustrated in Figs 9, 10 and 11. The structure is $0 \cdot 5$ mm long and has on it a nichrome aerial 20, feeding into a resistive load 21 consisting of a fine wire composed of nichrome $0 \cdot 5$ $\mu$m thick, 1 $\mu$m wide and 100 $\mu$m long which goes to and fro across a 20 $\mu$m square area, providing a resistive load of 200 ohms. The aerial, also being of nichrome, can be evaporated and patterned at the same time. When illuminated by microwaves at 10 GHz and with a rms electric field of 590 Vm$^{-1}$ (e = $13 \cdot 5$), roughly 80 microwatts will be dissipated in the load raising its temperature by about 20°C. This will melt a meltable plug 3 (eg of a hydrocarbon wax) on top of it, allowing the drug 1, enclosed between two impermeable layers 2a and 2b, to start dissolving out. Greater heating could be achieved using pulsed illumination, eg 10 $\mu$S on, 10 ms off. So long as the on time is long compared with the time constant for diffusion of heat from the wires, and the cycle time is short compared with the thermal time constant of the body, the mean power which can be safely dissipated is a constant (typically $1 \cdot 2$ kWm$^{-2}$ is a limitation in the body) but the temperature is proportional to the duty cycle ratio (in this case 1000 times which would yield 2000°C - enough not only to melt the plug but also to flash evaporate it and the nichrome).

The voltage generated is proportional to aerial length and the load impedance of a matched load is inversely proportional to it so the power available is proportional to length $^{-3}$ x illuminating frequency (assuming that the illuminsating frequency is still low compared with that at which the aerial is resonant). Alternatives to melting are the use of a suitable phase change or thermal expansion to rupture the containment and allow the drug to escape.

Structures of this type could be administered to a body in the manner of Example 2 or Example 3, and be set off on command by a burst of microwave radiation. A particular use, especially as the devices are made smaller, is to coat them with an antibody, allow some time for them to stick to a target tissue and then illuminate the area of interest within the body, causing those devices within this region to release their chemical. A minimum practicable size of device using microwave-based release is roughly 80 microns long.

In the case of larger devices of this nature, they could be implanted into the body as a contingency measure, eg against insulin deficiency or exposure to a poisons. When the situation arises a transmitter worn outside the body could be used to illuminate the devices and initiate release. In both these cases, both the ability to trigger the device on command, and the subsequent sustained release, are useful.

A further means of targeting, applicable both to this example and to Example 3, is to incorporate the particles into a phagocytic (eg a polymorphonuclear) cell by separating out cells from a sample of the patient's blood, allowing the cells to inject the devices and reinjecting them into the patient. The phagocytic cells will then migrate to the site of infection, such as an abcess. If the devices release their payload after a time delay of typically 4 hours, then most of the cells will by then have reached their target and the drug will have been applied selectively.

Example 9

The use of structures of the type shown in Example 1 to release corrosion-inhibiting additives into engine cooling water or engine oil eg in car and aero engines.

Example 10

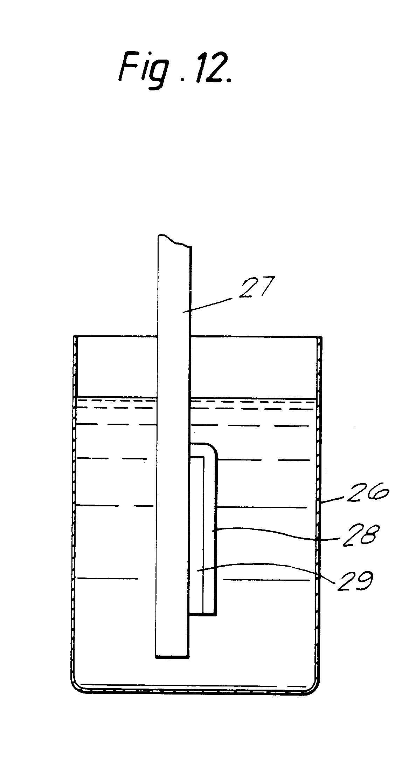

The use of the structure in chemical assays. Normally chemical assays are performed in a very rough and ready manner by means of indicator paper (eg pH paper) or more accurately by titration or by means of electrodes. In the latter cases the equipment is relatively large and/or fragile. These problems are overcome by placing a slow release structure in a known quantity of the material to be titrated, together with an end point detector (eg a glass electrode or methyl red or phenolphthalein for pH) and measuring the time taken for the end point to be reached. An example of the equipment required is shown in Fig 12. A container 26 has a small amount of indicator in it. It is then filled up to a mark with the material 27 to be titrated and a rod 28 with a slow release structure 29 on it containing the titrating agent and designed to release it at a well characterised, generally uniform rate, is placed in it. The rod can stir the solution as well as release material. The end point is timed and by reference to tables (generally requiring a temperature correction) or inspecting the slow release structure to see how far back it has dissolved, the concentration of material in the liquid can be determined.

Further, non-medical examples of applications of the invention include the following:

1. Antifoulants, especially for marine use, where they may be incorporated into paint and the chemical released at a sufficient rate to prevent the growth of barnacles and the like. This may require release sustained over a period of years. The frequency with which repainting is needed and the pollution due to excessive release when the paint is new are problems with anti-foulant paint currently available.

2. Other pesticides, including moluscicides, sustained-action herbicides, fungicides, algicides and insecticides. The ability to achieve pulsed release and the coordinated release of a plurality of materials is a major benefit in this context.

3. Release of preservatives in food.

4. Veterinary use in the control of growth through the steady release of steroids or antibiotics.

5. Release of nutrients for plants, microbial cultures and animals, including the release of trace elements in fertilisers.

## Claims

1. A method of fabricating a structure for the controlled release of a substance (1) located in the structure between two relatively insoluble layers (2a, 2b), characterised by the following stages:

   (a) depositing a first layer (2a) of a relatively insoluble material on a substrate (4) whose top layer at least is a substance which can be dissolved without affecting the materials of which the structure is made;

   (b) shaping the first layer of material (2a) by photolithography and etching;

   (c) depositing a layer of the substance to be released (1) on said first layer (2a);

   (d) shaping the layer of the substance (1) by photolithography and etching;

   (e) depositing a second layer (2b) of a relatively insoluble material over the layer of the substance (1);

   (f) shaping said second layer (2b) by photolithography and etching; and

   (g) removing the substrate (4) by dissolving at least the top layer thereof,

   the shapes of the layers (2a, 1, 2b) being determined so that the soluble layer (1) is entirely surrounded by the relatively insoluble layers (2a, 2b) except for one or more release orifices (3).

2. A method according to Claim 1 in which the soluble layer (1c) is shaped so that when being dissolved the cross-sectional area of the layer presented to the solvent, and hence the rate of dissolution, varies as dissolution progresses.

EP 0 239 605 B1

3. A method according to either preceding claim in which the soluble layer comprises a layer of the substance to be released (1a, 1b) and a layer (6) of a second soluble material, said layers being deposited one after another and not being co-extensive.

4. A method according to Claim 3 in which the layer of the substance to be released comprises discrete areas (1a, 1b) in contact with the second soluble material (6), whereby as the latter material dissolves a pulsed release of the said substance is obtained.

5. A method according to any preceding claim in which a plurality of different substances to be released (1a, 1b) are deposited in turn, whereby a combined or alternating release is obtained.

**Revendications**

1. Procédé de fabrication d'une structure de libération réglée d'une substance (1) placée dans la structure entre deux couches (2a, 2b) relativement insolubles, caractérisé par les étapes suivantes :
   (a) le dépôt d'une première couche (2a) d'une matière relativement insoluble sur un substrat (4) dont la couche supérieure au moins est formée d'une substance qui peut être dissoute sans affecter les matières dont est formée la structure,
   (b) la mise de le première couche de matière (2a) à une configuration voulue par photolithographie et attaque chimique,
   (c) le dépôt d'une couche de la substance à libérer (1) sur la première couche (2a),
   (d) la mise de la couche de la substance (1) à la configuration voulue par photolithographie et attaque chimique,
   (e) le dépôt d'une seconde couche (2b) d'une matière relativement insoluble sur la couche de la substance (1),
   (f) la conformation de la seconde couche (2b) par photolithographie et attaque chimique, et
   (g) l'enlèvement du substrat (4) par dissolution de sa couche supérieure au moins,
   les configurations des couches (2a, 1, 2b) étant déterminées afin que la couche soluble (1) soit totalement entourée par les couches relativement insolubles (2a, 2b), mis à part un ou plusieurs orifices de libération (3).

2. Procédé selon la revendication 1, dans lequel la couche soluble (1c) est mise à une forme telle que, lorsqu'elle est dissoute, la section de la couche présentée au solvant et en conséquence, la vitesse de dissolution varie lorsque la dissolution progresse.

3. Procédé selon l'une des revendications précédentes, dans lequel la couche soluble comprend une couche de la substance à libérer (1a, 1b) et une couche (6) d'une seconde matière soluble, les couches étant déposées l'une après l'autre et n'ayant pas la même étendue.

4. Procédé selon la revendication 3, dans lequel la couche de la substance qui doit être libérée comprend des zones séparées (1a, 1b) qui sont au contact de la seconde matière soluble (6), si bien que, lorsque cette dernière matière se dissout, une libération pulsée de la substance est obtenue.

5. Procédé selon l'une quelconque des revendications précédentes, dans lequel plusieurs substances différentes qui doivent être libérées (1a, 1b) sont déposées tour à tour, si bien qu'une libération combinée ou alternée est obtenue.

**Patentansprüche**

1. Verfahren zur Herstellung einer Struktur zur gesteuerten Freisetzung einer Substanz (1), die sich in der Struktur zwischen zwei relativ unlöslichen Schichten (2a, 2b) befindet, gekennzeichnet durch die folgenden Stufen:
   (a) Aufbringen einer ersten Schicht (2a) eines relativ unlöslichen Materials auf einem Substrat (4), dessen oberste Schicht mindestens eine Substanz ist, die gelöst werden kann, ohne die Materialien, aus denen die Struktur hergestellt ist, zu beeinträchtigen;
   (b) Formen der ersten Schicht aus dem Material (2a) durch Photolithographie und Ätzen;
   (c) Aufbringen einer Schicht der freizusetzenden Substanz (1) auf der ersten Schicht (2a);
   (d) Formen der Schicht aus der Substanz (1) durch Photolithographie und Ätzen;

8

(e) Aufbringen einer zweiten Schicht (2b) aus einem relativ unlöslichen Material über der Schicht der Substanz (1);

(f) Formen der zweiten Schicht (2b) durch Photolithographie und Ätzen; und

(g) Entfernen des Substrats (4) durch Auflösen mindestens seiner obersten Schicht,

wobei die Formen der Schichten (2a, 1, 2b) so festgelegt sind, daß die lösliche Schicht (1) von den relativ unlöslichen Schichten (2a, 2b) bis auf eine oder mehrere Freisetzungsöffnungen (3) vollständig umgeben ist.

2. Verfahren nach Anspruch 1, worin die lösliche Schicht (1c) so geformt ist, daß beim Lösen die dem Lösungsmittel ausgesetzte Querschnittsfläche der Schicht und damit die Geschwindigkeit der Auflösung mit dem Fortschreiten der Auflösung variiert.

3. Verfahren nach einem der beiden vorhergehenden Ansprüche, worin die lösliche Schicht eine Schicht der freizusetzenden Substanz (1a, 1b) und eine Schicht (6) eines zweiten löslichen Materials enthält, wobei die Schichten eine nach der anderen aufgebracht werden und nicht koextensiv sind.

4. Verfahren nach Anspruch 3, worin die Schicht der freizusetzenden Substanz getrennte Flächen (1a, 1b) besitzt, die in Kontakt stehen mit dem zweiten löslichen Material (6), wodurch, wenn sich das letztere Material auflöst, eine gepulste Freisetzung der Substanz erreicht wird.

5. Verfahren nach einem der vorhergehenden Ansprüche, worin eine Mehrzahl verschiedener freizusetzender Substanzen (1a, 1b) nacheinander aufgebracht werden, wodurch eine kombinierte oder alternierende Freisetzung erreicht wird.

Fig.1.

Fig.2.

Fig.3.

Fig.4.

Fig.5.

Fig.6.

Fig.7.

Fig.8.

# Fig . 9.

# Fig . 10.

# Fig .11.

# Fig .12.